# EUROPEAN PATENT SPECIFICATION

(11) **EP 2 265 111 B1**
(45) Date of publication and mention of the grant of the patent: **21.01.2015**
(21) Application number: 09720828.4
(22) Date of filing: 06.03.2009
(51) Int. Cl.: A01K 67/027, C12N 15/85

(54) **HAIRLESS IMMUNODEFICIENT MOUSE MODEL**
HAARLOSES IMMUNDEFIZIENTES MAUS-MODELL
MODÈLE DE SOURIS IMMUNODÉFICIENTE GLABRE

(30) Priority: 13.03.2008 US 47948
(43) Date of publication of application: 29.12.2010
(73) Proprietor: Charles River Laboratories, Inc., Wilmington, MA 01887-1000 (US)
(72) Inventor: CLIFFORD, Charles, B., Madison,WI 53703 (US); ELDER, Bruce J., North Andover, MA 01845 (US)
(74) Representative: Pohlman, Sandra M.
(86) International application number: PCT/US2009/036306
(87) International publication number: WO 2009/114410

(56) References cited:
- EP-A- 0 868 847
- EP-A- 1 859 676
- WO-A-00/40082
- WO-A-93/18144
- US-A- 5 859 307
- JIANMIN DUAN, ET AL.: "A novel model of HPV infection in meshed human foreskin grafts" ANTIVIRAL RESEARCHELSEVIER SCIENCE BV., AMSTERDAM, NL, vol. 64, no. 3, 7 October 2004 (2004-10-07), - December 2004 (2004-12) pages 179-188, XP004646260
- NONCHEV, S., BRANCAZ, M.V. FOLCO, E., ROMERO, Y. IRATNI, R.: "Le gène hairless de la souris" MÉDECINE SCIENCES, vol. 22, no. 5, May 2006 (2006-05), pages 525-530, XP002529660
- THOMPSON CC, SISK JM, BEAUDOIN GM 3RD.: "Hairless and Wnt signaling: allies in epithelial stem cell differentiation." CELL CYCLE., vol. 5, no. 17, 1 September 2006 (2006-09-01), XP002529039
- M J BOSMA, AND A M CARROLL: "The Scid Mouse Mutant: Definition, Characterization, and Potential Uses" ANNUAL REVIEW OF IMMUNOLOGY, vol. 9, April 1991 (1991-04), pages 323-350, XP002529661 cited in the application
- NIEDERKORN J Y; UBELAKER J E; MARTIN J M: "VASCULARIZATION OF CORNEAS OF HAIRLESS MUTANT MICE" INVESTIGATIVE OPHTHALMOLOGY AND VISUAL SCIENCE, vol. 31, no. 5, 1990, pages 948-953, XP002529662

## Description

### RELATED APPLICATIONS

### FIELD OF THE INVENTION

The field of the invention is medical research, immunology, and oncology, and, in particular, the development of a mouse model.

### BACKGROUND

Immunodeficient mouse models are useful in medical research, particularly oncology and immunology research, because an immunodeficient recipient mouse can receive foreign tissue and tumor transplants, called xenografts, without eliciting an immune response rejecting the foreign tissue or tumor. The immunodeficient mouse model most commonly used in xenograft studies is the athymic nude mouse. Nude mice homozygous for a recessive *Foxn1^{nu}* mutation are T-cell deficient (thus are immunodeficient) and lack a hair coat. Nude mice heterozygous for the *Foxn1^{nu}* mutation, however, are immunocompetent and have a normal coat of hair. Another commonly used immunodeficient mouse model is known as the NIH III mouse model, in which the mice are T-cell, B-cell, and natural killer cell deficient, and used for example in WO00/40082 as a hairless immunodeficient skin xenograft mouse model for producing human papillomoviruses (HPV).

The breeding of nude mice presents certain challenges. Homozygous female nude mice cannot be used for breeding because of poor reproductive performance due to an irregular estrous cycle and limited lactation capacity resulting in non-viable offspring. Therefore, the standard breeding practice for nude mice is to mate a homozygous male and heterozygous female. One half of the resulting offspring are homozygous for a recessive *Foxn1^{nu}* mutation, and therefore immunodeficient, and lacking a hair coat. The other half of the offspring are heterozygous for a recessive *Foxn1^{nu}* mutation, and thus immunocompetent, and haired. As such, half of the animals produced by the standard breeding practice of nude mice do not fit the immunodeficiency requirements for xenograft studies and, therefore, are unusable for these studies. Similar breeding challenges also exist for NIH III mice. Thus, the breeding of athymic nude mice and NIH III mice is inefficient as one half of the progeny are generally not used in medical research. As a result, there remains a need for an efficient breeding protocol to produce immunodeficient hairless mice.

### SUMMARY

The present invention is based, in part, upon the discovery that a mouse homozygous for a recessive hairless *hr* allele (*Hr^{hr}*) can be bred with an immunodeficient mouse that is homozygous for a recessive Prkdc^{scid} allele which thereby disrupts differentiation of both B lymphocyte progenitor cells and T lymphocyte progenitor cells to produce a hairless, immunodeficient mouse that is homozygous at both the hairless Hr^{hr} allele and the Prkdc^{scid} allele resulting in the B-cell deficiency and T-cell deficiency. The hairless, immunodeficient mice described herein offer an advantage over both the athymic mouse model and NIH III mouse model because these mice can be maintained as homozygous breeding pairs. As a result, all of the progeny from these breeding pairs can be used for xenograft studies and, therefore, the number of animals that cannot be used for xenograft studies is significantly reduced. In addition, the hairless, immunodeficient mice described herein comprise both a B-cell deficiency and a T-cell deficiency and, therefore, are more severely immunocompromised than athymic nude mice allowing a higher percentage of engraftment, increased tumor growth rate, and less tumor regression. The hairless mice are particularly useful as the xenografts can usually be identified visually without the need for palpation. In contrast, when the animals have a hair coat, the xenografts usually need to be identified by touch.

The present invention discloses a hairless, immunodeficient mouse that is homozygous for a recessive *hr* allele (*Hr^{hr}*) and further is homozygous for a recessive scid allele (*Prkdc^{scid}*) which thereby disrupts differentiation of both B lymphocyte progenitor cells and T lymphocyte progenitor cells. The hairless immunodeficient mouse may also be homozygous for a recessive beige allele (*Lyst^{bg}*)

The present invention also provides methods for producing a hairless, immunodeficient mouse comprising breeding a first mouse strain homozygous for a recessive hairless (*hr*) allele (*Hr^{hr}*) with a second, different mouse strain homozygous for a recessive Prkdc^{scid} allele, which disrupts differentiation of both B lymphocyte progenitor cells and T lymphocyte progenitor cells. Progeny from the F1 cross are heterozygous for both the Hr^{hr} allele and the Prkdc^{scid} allele disrupting differentiation of both B lymphocyte progenitor cells and T lymphocyte progenitor cells. The heterozygous progeny then are intercrossed and homozygous hairless Hr^{hr} progeny are selected. Hairless homozygous *hr* progeny from the first intercross are either homozygous or heterozygous for the Prkdc^{scid} allele disrupting differentiation of both B lymphocyte progenitor cells and T lymphocyte progenitor cells, and are intercrossed to obtain progeny homozygous at both the Hr^{hr} allele and the Prkdc^{scid} disrupting differentiation of both B lymphocyte progenitor cells and T lymphocyte progenitor cells. Homozygosity at both mutations may be confirmed by genetic testing methods well known in the art.

These and other aspects and advantages of the invention will become apparent upon consideration of the following detailed description and claims.

### DETAILED DESCRIPTION

The present invention discloses hairless, immunodeficient mouse models that are homozygous for a recessive *hr* allele (*Hr^{hr}*) and further homozygous for the Prkdc^{scid} allele, which results in both a B-cell deficiency and a T-cell deficiency. The hairless, immunodeficient mouse models described herein offer a significant advantage over the athymic nude mouse model and the NIH III mouse model because they can be maintained using homozygous breeding pairs. As a result, all of the progeny obtained from the homozygous breeding pairs are hairless and comprise both a B-cell deficiency and a T-cell deficiency. The ability to breed and maintain hairless, immunodeficient mouse colonies significantly reduces the number of animals, which are produced and not useable in medical research for their intended purpose. Further, the hairless, immunodeficient mouse models described herein are more severely immunocompromised than the athymic nude mice. The greater immunodeficiency of the mouse models of the invention allow for better growth of xenogenic tumor lines. Furthermore, hairless mice also allow rapid assessment of cutaneous and subcutaneous tissue and tumor grafts.

The disclosure also provides a hairless, immunodeficient mouse having a hairless Hr^{hr} allele and further comprising a Prkdc^{scid} allele that disrupts differentiation of both B lymphocyte progenitor cells and T lymphocyte progenitor cells.

The hairless, immunodeficient mice homozygous for both the recessive *hr* allele (*Hr^{hr}*) and the recessive scid allele (*Prkdc^{scid}*) are phenotypically characterized as hairless, B-cell deficient, and T-cell deficient. Hairless, scid mice are further characterized as maintaining normal natural killer cells, macrophages, and granulocytes, and possess lymph nodes and a thymus. Under certain circumstances, in one embodiment it is desirable to produce mice homozygous for a recessive *hr* allele (*Hr^{hr}*) and homozygous for a scid allele (*Prkdc^{scid}*) that are also homozygous for a recessive beige allele (*Lyst^{bg}*). Mice homozygous for the hairless, scid, and beige alleles are phenotypically characterized as hairless, B-cell deficient, T-cell deficient, have granulocytes that are defective and have reduced activity and a severe deficiency of natural killer cells.

In another aspect, the invention provides a method for producing a hairless, immunodeficient mouse. The method comprises the steps of (a) crossing a first mouse strain homozygous for a recessive Hr^{hr} allele with a second, different mouse strain homozygous for a Prkdc^{scid} allele that disrupts differentiation of both B lymphocyte progenitor cells and T lymphocyte progenitor cells to produce progeny heterozygous for both mutations; (b) intercrossing the heterozygous progeny produced by step (a); (c) selecting hairless progeny produced by step (b) homozygous for the Hr^{hr} allele; and (d) intercrossing hairless mice homozygous or heterozygous for Prkdc^{scid} allele resulting in a B-cell deficiency and T-cell deficiency to obtain progeny homozygous for both mutations.

Hairless progeny homozygous for the recessive Hr^{hr} allele can be selected based on phenotypic characterization. Mice homozygous for the Hr^{hr} allele develop a hair coat after birth, but lose their hair coat within one-month of birth, for example, within about 20 days of birth, within about 25 days of birth, or within about 30 days (about one-month) of birth. Mice homozygous for the recessive Hr^{hr} allele may be selected based on the loss of their hair coat prior to or at weaning or within one-month of birth. Mice heterozygous for the recessive Hr^{hr} allele do not lose their hair coat. Hairless progeny homozygous for the recessive Hr^{hr} allele may also be selected by genotypic testing methods well known in the art, including, for example, tail biopsies and ear clippings.

In some embodiments, the method for producing a hairless, immunodeficient mouse may further comprise the step of determining the zygosity of the Prkdc^{scid} disrupting the differentiation of both B lymphocyte progenitor cells and T lymphocyte progenitor cells in the hairless progeny selected in step (c). Zygosity of the recessive Prkdc^{scid} resulting in both a B-cell deficiency and T-cell deficiency may be determined by genetic testing methods well known in the art, including, for example, tail biopsies and ear clippings.

In another embodiment, the method for producing a hairless, immunodeficient mouse may further comprise the step of, after step (d), confirming that the progeny are homozygous for the recessive Hr^{hr} allele and homozygous for the recessive Prkdc^{scid} disrupting differentiation of both B lymphocyte progenitor cells and T lymphocyte progenitor cells. Zygosity of both mutations may be confirmed by genetic testing methods well known in the art, including, for example, tail biopsies and ear clippings.

Exemplary hairless mice that can be used to produce the hairless immunodeficient mice of the invention are homozygous for the recessive Hr^{hr} allele and are characterized as euthymic and immunocompetent. One such mouse useful in making the hairless models described herein is the Cr1:SKH1-*Hr^{hr}* mouse strain that can be obtained from Charles River Laboratories, Wilmington, MA.

Exemplary mice having one or more further mutations that disrupt the differentiation of both B and T lymphocyte progenitor cells that can be used to produce the immunodeficient mice of the invention include mice having one further mutation in the beige allele. Mice having one further mutation in the beige allele may be maintained as inbred or outbred lines.

Scid mice from the second mouse strain homozygous for a recessive scid allele (*Prkdc^{scid}*) possess an autosomal recessive mutation in the *Prkdc* gene and are characterized as having a severe combined immunodeficiency disrupting differentiation of both B lymphocyte progenitor cells and T lymphocyte progenitor cells. Scid mice lack dendritic Thy-1+ epidermal cells and this deficiency results in their inability to produce antibodies and to reject allogenic and xenogenic tissue and tumor grafts. Scid mice are further characterized as maintaining normal natural killer cells, macrophages, and granulocytes, and possess lymph nodes and a thymus. Scid mice, for example, the Crl:HA- *Prkdc^{scid}* mouse strain, can be obtained from Charles River Laboratories, Wilmington, MA. In another embodiment, the mouse strain homozygous for a recessive scid allele is additionally homozygous for a recessive beige allele (*Lyst^{bg}*). SCID Beige mice possess autosomal recessive mutations in both the *Prkdc* gene and the *Lyst* gene. In addition to lacking functional B lymphocytes and T lymphocytes, beige mice also lack natural killer (NK) cells and are characterized as B-cell deficient, T-cell deficient, and NK cell deficient. Beige mice, for example, the CB17.B6-*Prkdc^{scid} Lyst^{bg}* mouse strain, can be obtained from Charles River Laboratories, Wilmington, MA.

A hairless, immunodeficient mouse homozygous for a recessive *hr* allele (*Hr^{hr}*) and homozygous for a recessive scid allele (*Prkdc^{scid}*) can be produced by crossing a hairless mouse with a scid mouse. An exemplary hairless mouse strain is Cr1:SKH1-*Hr^{hr}* and an exemplary scid mouse strain is Cr1:HA- *Prkdc^{scid}.* The progeny resulting from the F1 cross are heterozygous for the recessive *hr* allele and the recessive scid allele. The heterozygous progeny then can be intercrossed to begin returning the mutations to the homozygous state. Hairless F2 mice then are selected based on phenotype, i.e., the loss of their hair coat. Tail biopsies can be collected at weaning from the hairless mice and submitted for genetic testing to determine the zygosity of the scid mutation. Mice that are either homozygous or heterozygous for the scid mutation can be selected for mating to produce F3 progeny. All mice progeny are hairless, and tail biopsies are taken from all mice to determine zygosity of the scid allele. Double homozygous mice are selected to build a mouse colony of hairless-scid mice.

Under certain circumstances, it is beneficial for the scid mouse to also be homozygous for a recessive beige allele (*Lyst^{bg}*). An exemplary scid-beige mouse strain is CB17.B6-*Prkdc^{scid} Lyst^{bg}.* The animals can be produced essentially as described above. The F2 progeny are selected based on phenotype, i.e., the loss of their hair coat. Tail biopsies are collected at weaning from the hairless mice and submitted for genetic testing to determine the zygosity of the scid allele. Blood smears can be used to identify animals homozygous for the beige mutation. Mice that are either homozygous or heterozygous for the scid mutation and homozygous for the beige mutation are selected for mating to produce F3 progeny. Triple homozygous mice are selected to build a colony of hairless scid-beige mice.

The hairless, immunodeficient mouse model described herein can be maintained as a mouse colony by breeding male and female mice each of which are mouse homozygous for a recessive *hr* allele (*Hr^{hr}*) and homozygous for the recessive Prkdc^{scid} allele. The ability to breed hairless, immunodeficient male mice with hairless, immunodeficient female mice produces progeny that are all hairless and immunodeficient.

The hairless, immunodeficient mouse model described herein comprises both a B-cell deficiency and a T-cell deficiency. As a result, this mouse model is susceptible to infection from a variety of organisms, which would not otherwise pose a threat to an immunocompetent mouse. In addition to mortality and/or clinical illness, such an infection may induce responses in the remaining host-defense mechanisms of the hairless, immunodeficient mouse that inhibit growth of implanted tumors. Further, xenograft studies in the hairless, immunodeficient mice described herein may include the use of chemotherapeutic compounds. Chemotherapeutic compounds may suppress the production of leukocytes, such as neutrophils, which play key role in the rapid immune response to bacterial challenge, emphasizing the need for mice free of specified bacteria and other infectious organisms, examples of which are described herein. Thus, hairless, immunodeficient mice described herein preferably are bred, housed, and transported in a manner that excludes pathogens, for example, pathogenic bacteria, fungi, parasites and viruses. Accordingly, the animals are bred, housed, transported, and otherwise maintained under conditions to be pathogen free.

In certain embodiments, the hairless, immunodeficient mice described herein are free of pathogens (for example, pathogenic bacteria, viruses, fungi or parasites) that cause clinical disease or subclinical infections. In one embodiment, the hairless, immunodeficient mice described herein are free from viruses that may cause clinical disease or subclinical infections, including the viruses selected from the group consisting of mouse rotavirus (EDIM), Sendai (SEND), mouse cytomegalovirus (MCMV), mouse hepatitis virus (MHV), mouse norovirus (MNV), mouse parvoviruses (MPV1-4), minute virus of mice (MVM), mouse thymic virus (MTLV), Theiler's murine encephalomyelitis virus (TMEV), K virus (K), mousepox (Ectromelia), lactate dehydrogenase-elevating virus (LDV), Hantaviruses, pneumonia virus of mice (PVM), reovirus types 1 and 3 (REO), mouse adenovirus (MAV-1 and MAV-2), lymphocytic choriomeningitis virus (LCMV), and polyoma virus (POLY).

In another embodiment, the hairless, immunodeficient mice described herein are free from bacteria that may cause clinical disease or subclinical infections, including the bacteria selected from the group consisting of *Salmonella,* CAR bacillus, *Helicobacter* (including all *Heliobacter* species), *Clostridium piliforme, Corynebacterium kutscheri, Corynebacterium bovis, Citrobacter rodentium, Mycoplasma pulmonis* and other species, *Streptobacillus moniliformis, Bordetella bronchiseptica,* Beta-hemolytic streptococci, *Streptococcus pneumoniae, Staphylococcus aureus,* other coagulase-positive staphylococci, *Pasteurella pneumotropica, Pseudomonas* (all species), and *Klebsiella* (all species).

In another embodiment, the hairless, immunodeficient mice described herein are free from fungi that may cause clinical disease or subclinical infections, including the fungi selected from the group consisting of *Encephalitozoon cuniculi* (ECUN) and *Pneumocystis.*

In another embodiment, the hairless, immunodeficient mice described herein are free from parasites that may cause clinical disease or subclinical infections, including the parasites selected from the group consisting of external and internal multicellular and unicellular parasites.

In certain embodiments, the hairless, immunodeficient mice are tested for a panel of infectious organisms that may cause clinical disease or subclinical infections including, for example, mouse rotavirus (EDIM), Sendai (SEND), mouse cytomegalovirus (MCMV), mouse hepatitis virus (MHV), pneumonia virus of mice (PVM), *Encephalitozoon cuniculi* (ECUN), mouse parvoviruses (MPV1-4), minute virus of mice (MVM), K virus (K), lactate dehydrogenase-elevating virus (LDV), Hantaviruses, reovirus types 1 and 3 (REO), mouse adenovirus (MAV-1 and MAV-2), lymphocytic choriomeningitis virus (LCMV), polyoma virus (POLY), Theiler's murine encephalomyelitis virus (TMEV, GD-7), Ectromelia (Ectro), CAR bacillus, *Clostridium piliforme, Corynebacterium kutscheri, Mycoplasma pulmonis, Bordetella bronchiseptica, Citrobacter rodentium, Helicobacter hepaticus, Salmonella* species, *Streptobacillus moniliformis, Pneumocystis carinii,* β-hemolytic *Streptococcus* species, coagulase (+) *Staphylococcus* species, *Corynebacterium bovis, Pasteurella pneumotropica, Streptococcus pneumoniae, Mycoplasma* species, *Helicobacter bilis, Helicobacter* species, coagulase (-) *Staphylococcus* species, non-β-hemolytic *Streptococcus* species, *Klebsiella* species, *Pseudomonas aeruginosa, Corynebacterium* species, and *Bacillus* species.

### EXEMPLIFICATION

The following Examples discuss the production and characterization of the hairless, immunodeficient mouse models described herein.

### EXAMPLE 1: Hairless-Scid Mouse Model

A hairless, immunodeficient mouse homozygous for a recessive *hr* allele (*Hr^{hr}*) and homozygous for a recessive scid allele (*Prkdc^{scid}*) was generated by crossing a male hairless mouse (strain Cr1:SKH1-*Hr^{hr}*) with a female scid mouse (strain Cr1:HA- *Prkdc^{scid}*). Prior to crossing the two lines, each one was maintained as a genetically outbred stock. The progeny resulting from the F1 cross were heterozygous for the recessive *hr* allele and the recessive scid allele. The heterozygous progeny then were intercrossed to begin returning the mutations to the homozygous state. Hairless F2 mice were selected based on phenotype, i.e., the loss of their hair coat. Tail biopsies were collected at weaning from the hairless mice and submitted for genetic testing to determine the zygosity of the scid mutation. Mice that were either homozygous or heterozygous for the scid mutation were selected for mating to produce F3 progeny. All mice produced were hairless, and tail biopsies were taken from all mice to determine zygosity of the scid allele. Double homozygous mice were selected to build a mouse colony of hairless-scid mice.

## Claims

1. A hairless, immunodeficient mouse having a *hr* allele (*Hr^{hr}*) and both a B-cell deficiency and a T-cell deficiency, wherein the mouse is homozygous for the recessive *hr* allele (*Hr^{hr}*) and homozygous for a recessive *Prkdc^{scid}* allele, which thereby disrupts differentiation of both B lymphocyte progenitor cells and T lymphocyte progenitor cells.

2. The mouse of claim 1, wherein the mouse is also homozygous for a recessive beige allele (*Lyst^{bg}*).

3. The mouse of claims 1 or 2, wherein the mouse has hair after birth, but then loses its hair within one month after birth.

4. A method for producing a hairless, immunodeficient mouse, the method comprising the steps of:
(a) crossing a first mouse strain homozygous for a recessive *Hr^{hr}* allele with a second, different mouse strain homozygous for a recessive *Prkdc^{scid}* allele to produce progeny heterozygous for both the *Hr^{hr}* allele and the *Prkdc^{scid}* allele;
(b) intercrossing the heterozygous progeny produced by step (a);
(c) selecting hairless progeny produced by step (b) homozygous for the *Hr^{hr}* allele; and
(d) intercrossing hairless mice homozygous or heterozygous for the *Prkdc^{scid}* allele to obtain progeny homozygous for both the *Hr^{hr}* allele and the *Prkdc^{scid}* allele.

5. The method of claim 4, further comprising the step of determining the zygosity of the *Prkdc^{scid}* allele in the hairless progeny selected in step (c).

6. The method of claim 5, wherein the zygosity of the *Prkdc^{scid}* allele is determined by genetic testing.

7. The method of any one of claims 4-6, wherein, in step (a), the first mouse strain is a CrL:SKH1-*HR^{hr}* mouse.

8. The method of any one of claims 4-7, wherein, in step (a), the second mouse strain is a Crl:HA-*Prkdcs^{scid}* mouse.

9. The method of any one of claims 4-8, further comprising the step of, after step (d), confirming that the progeny are homozygous for the recessive *Hr^{hr}* allele and homozygous for the recessive *Prkdc^{scid}* allele.

10. A colony of mice of any one of claims 1-3.

11. A colony of mice produced by the method of any one of claims 4-9.

## Patentansprüche

1. Haarlose, immundefiziente Maus, welche ein *hr*-Allel (*Hr^{hr}*) und sowohl eine B-Zell-Defizienz als auch eine T-Zell-Defizienz aufweist, wobei die Maus homozygot für das rezessive *hr*-Allel (*Hr^{hr}*) und homozygot für ein rezessives *Prkdc^{scid}*-Allel ist, welche dadurch die Differenzierung von sowohl B-Lymphozyten-Vorläuferzellen als auch T-Lymphozyten-Vorläuferzellen stört.

2. Maus nach Anspruch 1, wobei die Maus auch homozygot für ein rezessives Beige-Allel (*Lysf^{bg}*) ist.

3. Maus nach Anspruch 1 oder 2, wobei die Maus nach der Geburt Fell aufweist, jedoch ihr Fell dann innerhalb eines Monats nach der Geburt verliert.

4. Verfahren zum Erzeugen einer haarlosen, immundefizienten Maus, wobei das Verfahren folgende Schritte umfasst:
(a) Kreuzen eines ersten Mausstammes, welcher homozygot für ein rezessives *Hr^{hr}*-Allel ist, mit einem zweiten, unterschiedlichen Mausstamm, welcher homozygot für ein rezessives *Prkdc^{scid}*-Allel ist, um Nachkommen zu erzeugen, die heterozygot für sowohl das *Hr^{hr}*-Allel als auch das *Prkdc^{scid}*-Allel sind;
(b) Kreuzen zwischen den heterozygoten Nachkommen erzeugt durch Schritt (a);
(c) Auswählen haarloser Nachkommen erzeugt durch Schritt (b), welche homozygot für das *Hr^{hr}*-Allel sind;
und
(d) Kreuzen zwischen haarlosen Mäusen, welche homozygot oder heterozygot für das *Prkdc^{scid}*-Allel sind, zum Erhalten von Nachkommen, welche homozygot für sowohl das *Hr^{hr}*-Allel als auch das *Prkdc^{scid}*-Allel sind.

5. Verfahren nach Anspruch 4, welches ferner den Schritt des Bestimmens der Zygotie des *Prkdc^{scid}*-Allels bei den haarlosen Nachkommen ausgewählt in Schritt (c) umfasst.

6. Verfahren nach Anspruch 5, wobei die Zygotie des *Prkdc^{scid}*-Allels durch Gentests bestimmt wird.

7. Verfahren nach einem der Ansprüche 4-6, wobei, in Schritt (a), der erste Mausstamm eine Crl:SKH1-*Hr^{hr}*-Maus ist.

8. Verfahren nach einem der Ansprüche 4-7, wobei, in Schritt (a), der zweite Mausstamm eine Crl:HA-*Prkdc^{scid}*-Maus ist.

9. Verfahren nach einem der Ansprüche 4-8, welches ferner, nach Schritt (d), den Schritt des Bestätigens umfasst, dass die Nachkommen homozygot für das rezessive *Hr^{hr}*-Allel und homozygot für das rezessive *Prkdc^{scid}*-Allel sind.

10. Mauskolonie nach einem der Ansprüche 1-3.

11. Mauskolonie nach dem Verfahren von einem der Ansprüche 4-9 hergestellt.

## Revendications

1. Souris immunodéficiente glabre possédant un allèle *hr* (*Hr^{hr}*) et à la fois une déficience en cellules B et une déficience en cellules T, dans laquelle la souris est homozygote pour l'allèle *hr* récessif (*Hr^{hr}*) et homozygote pour un allèle *Prkdc^{scid}* récessif, qui ainsi perturbe la différenciation à la fois des cellules progénitrices de lymphocyte B et des cellules progénitrices de lymphocyte T.

2. Souris selon la revendication 1, dans laquelle la souris est également homozygote pour un allèle beige récessif (*Lyst^{bg}*).

3. Souris selon la revendication 1 ou la revendication 2, dans laquelle la souris possède des poils après la naissance, mais perd ensuite ses poils dans le mois suivant la naissance.

4. Procédé de production d'une souris immunodéficiente glabre, le procédé comprenant les étapes consistant à :
(a) croiser une première souche de souris homozygote pour un allèle *Hr^{hr}* récessif avec une deuxième souche différente de souris homozygote pour un allèle scid récessif pour produire une progéniture hétérozygote à la fois pour l'allèle *Hr^{hr}* et l'allèle *Prkdc^{scid}* ;
(b) inter-croiser la progéniture hétérozygote produite par l'étape (a) ;
(c) sélectionner la progéniture glabre produite par l'étape (b) homozygote pour l'allèle *hr ;* et
(d) inter-croiser des souris glabres homozygotes ou hétérozygotes pour l'allèle *Prkdc^{scid}* pour obtenir une progéniture homozygote à la fois pour l'allèle *Hr^{hr}* et l'allèle *Prkdc^{scid}*.

5. Procédé selon la revendication 4, comprenant en outre l'étape consistant à déterminer la zygosité de l'allèle scid dans la progéniture glabre sélectionnée à l'étape (c).

6. Procédé selon la revendication 5, dans lequel la zygosité de l'allèle scid est déterminée par test génétique.

7. Procédé selon l'une quelconque des revendications 4 à 6, dans lequel, à l'étape (a), la première souche de souris est une souris Crl:SKH1-*Hr^{hr}*.

8. Procédé selon l'une quelconque des revendications 4 à 7, dans lequel, à l'étape (a), la deuxième souche de souris est une souris Crl:HA-*Prkdc^{scid}*.

9. Procédé selon l'une quelconque des revendications 4 à 8, comprenant en outre, après l'étape (d), l'étape consistant à confirmer que la progéniture est homozygote pour l'allèle *Hr^{hr}* récessif et homozygote pour l'allèle *Prkdc^{scid}* récessif.

10. Colonie de souris selon l'une quelconque des revendications 1 à 3.

11. Colonie de souris produit par le procédé selon l'une quelconque des revendications 4-9.
